## Europäisches Patentamt
## European Patent Office
## Office européen des brevets

(11) Numéro de publication: **0 185 123**
**B1**

(12) **FASCICULE DE BREVET EUROPEEN**

(45) Date de publication du fascicule du brevet: **26.10.88**

(21) Numéro de dépôt: **84402542.9**

(22) Date de dépôt: **10.12.84**

(51) Int. Cl.⁴: **A 61 F 13/00,** A 61 H 7/00, A 41 B 11/00

(54) **Procédé de traitement des membres inférieurs.**

(43) Date de publication de la demande: **25.06.86 Bulletin 86/26**

(45) Mention de la délivrance du brevet: **26.10.88 Bulletin 88/43**

(84) Etats contractants désignés: **BE DE IT**

(56) Documents cités:
**CH-A- 377 980**
**FR-A-2 213 762**
**FR-A-2 432 867**
**US-A-3 028 857**
**US-A-4 240 160**

(73) Titulaire: **Delille, Adrienne**
**87, rue Saint-Lazare**
**F-75009 Paris (FR)**

(72) Inventeur: **Delille, Adrienne**
**87, rue Saint-Lazare**
**F-75009 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Description

La présente invention se rapporte à un procédé de traitement des membres inférieurs. CH—A—377980 décrit un bas pour soins des jambes, destiné à être imprégné de produit liquide. US—A—4 240 160 décrit un collant à varices ayant une contention dégressive vers le haut. La présente invention a pour objet la potentialisation de l'effet d'une crème de soins par le port d'un collant de contention.

A cet effet et conformément à l'invention il est proposé un procédé pour masser les membres inférieurs excluant les méthodes thérapeutiques, ledit procédé comprenant l'application d'une crème de traitement concomitamment au port d'un collant ayant une contention dégréssive vers le haut du membre pendant la marche avec le collant.

La crème employée en massages circulaires est potentialisée par le port du collant masseur à contention dégréssive vers le haut. Ce procédé pour masser les membres inférieurs employant ensemble une crème de traitement et le port d'un collant à contention dégréssive vers le haut facilitant la circulation de retour pendant la marche est un procédé de traitement efficace et économique pour les membres inférieurs.

## Revendication

Procédé pour masser les membres inférieurs excluant les méthodes de traitement thérapeutique, ledit procédé comprenant l'application d'une crème de traitement concomitamment au port d'un collant ayant une contention dégréssive vers le haut du membre pendant la marche avec le collant.

## Patentanspruch

Massageverfahren der unteren Gliedmassen, das keine Therapie erforderlich macht, da bei diesem Verfahren eine Behandlungssalbe, die auf einen Stützstrumpf mit nach oben hin abnehmender Retention aufgetragen wird, beim Gehen auf das Bein einwirkt.

## Claim

Technique to massage the lower limbs precluding therapeutic treatment methods; the said technique involves the application of a treatment cream together with the wearing of a stocking which relieves pressure upwards in the limb while walking with the stocking.